# EUROPEAN PATENT SPECIFICATION

(11) **EP 3 475 013 B1**
(45) Date of publication and mention of the grant of the patent: **21.04.2021**
(21) Application number: 17821152.0
(22) Date of filing: 28.06.2017
(51) Int. Cl.: B22F 1/00, B22F 3/12

(54) **METHODS OF FORMING AN OXIDE LAYER ON A METAL BODY**
VERFAHREN ZUR HERSTELLUNG EINER OXIDSCHICHT AUF EINEM METALLKÖRPER
PROCÉDÉS DE FORMATION D'UNE COUCHE D'OXYDE SUR UN CORPS MÉTALLIQUE

(30) Priority: 28.06.2016 US 201615195624
(43) Date of publication of application: 01.05.2019
(73) Proprietor: Viper Technologies LLC, Portland, OR 97230 (US)
(72) Inventor: WALKER, David, L., deceased (US)
(74) Representative: Samson & Partner Patentanwälte mbB
(86) International application number: PCT/US2017/039761
(87) International publication number: WO 2018/005650

(56) References cited:
- US-A1- 2002 042 656
- US-A1- 2006 285 991
- US-A1- 2012 136 400
- US-A1- 2015 107 721

## Description

### Background

The present disclosure relates generally to methods for forming an oxide layer on metal bodies. In particular, methods of forming an oxide layer on a zirconium alloy surface of a metal body are described.

Known methods of producing zirconium alloy based medical implants are not entirely satisfactory for the range of applications in which they are employed. Zirconium alloys, and Zirconium-2.5 Niobium in particular, has been used to in medical implants for weight bearing components, such as femoral heads and knee replacement femoral components. One benefit of using zirconium alloy is that an oxidation process can be readily applied to create a substantially dense, smooth, and uniform layer of zirconium oxide, which acheives a low friction coefficient and is exceptionally hard.

An oxidation layer provides numerous operational benefits, such as superior resistance to wear and corrosion. These specific benefits are particularly relevant in the medical implant industry because malfunctioning implants could cause bodily injury or require invasive surgery to repair. The low friction coefficient of oxide layers tend to reduce wear when the oxide surfaces articulate against relatively soft materials, such as plastic implants paired with metal implants.

Traditional cast zirconium alloy parts may not be suitable for medical applications. For example, many cast zirconium alloy parts have had unsatisfactory surface conditions to enable a satisfactory zirconium oxide layer to be formed on the surface. Specifically, many cast zirconium alloy products define ]arge, visible grain boundaries, which often result in uneven and cracked zirconium oxide layers when the products are oxidized. Further, these imperfections often result in a product that visually appears unreliable, which may lower medical practitioners' confidence in them. Furthennore, solid zirconium alloy bodies may not achieve the necessary strength to weight ratio required in some situations.

Each of these limitations of conventional zirconium alloy casting processes result in lower quality products. The lower quality products typically have less dense, smooth, and uniform surfaces and lack the resistance to corrosion and wear of forged products. As a result, conventional zirconium alloy parts are not a cost-effective, suitable replacements for forged zirconium alloy parts.

Regarding traditional cast zirconium alloy bodies US 2015/0107721 A1 discloses methods of preparing a surface of a cast zirconium alloy body for oxidation, the method including hot isostatic pressing a cast body of near shape dimensions, heating the cast body, machining the cast body to desired shape dimensions, and treating the surface of the cast body to accept an oxide layer.

US 2012/0136400 A1 discloses a metal injection molding process comprising the steps of molding two separate components from feedstock comprising a material powder and a binder, assembling the two components, removing the binder and performing a sintering operation on the assembled component to bond the two components together.

Thus, there exists a need for processes for producing zirconium alloy products that that improve upon and advance the design of known methods. In particular, the field requires methods to increase the quality of the zirconium oxide layer of metal bodies to be used, for example, as medical implants. Even more particularly, there exists a need for methods of preparing the surface of cast zirconium alloy products with improved surface characteristics prior to creating the oxidized layer.

Furthermore, traditional casting processes may not allow for the incorporation of substructures of different compositions within the metal body. Thus, there exists a need for methods of producing a zirconium oxide layer an metal bodies produced by methods other than casting.

It would be desirable to increase the quality of the oxidized layer of products having a zirconium-containing surface. Such products may be a cost-effective and suitable replacement of, for example, forged zirconium alloy products. Indeed, it would be desirable to produce high-quality zirconium alloy products at a significantly lower price than conventionalforged products. Examples of nerv and useful methods relevant to increasing the quality of suchzirconium-containing products are described below.

### Summary

A method of forming an oxide layer on a surface of a Metal Injection Molded (MIM) body according to the invention is defined in claim 1.

In an example also disclosed, a method of producing a oxide layer on metal product may include preparing a surface of a cast zirconium alloy substrate for oxidation, hot isostatic pressing a cast substrate of near shape dimensions, heating the cast substrate, machining the cast substrate to desired shape dimensions, and treating the surface of die cast substrate to accept an oxide layer. In some examples, treating die surface of the cast substrate may include polishing the surface, peening the polished surface, and finishing the peened surface. Additional or alternative examples may include heat treating a cast substrate of near shape dimensions to define a homogenized grain structure within the cast substrate, machining the heat treated cast substrate to desired shape dimensions, and surface treating the machined cast substrate to modify its structure to define a recrystallized modified grain structure defining a reduced grain boundary size.

### Brief Description of the Drawings

Fig. 1 is a flow diagram depicting a first example of a method of forming an oxidized surface on a cast zirconium alloy substrate.
Fig. 2 is bottom perspective view of a first example of a cast zirconium alloy substrate defining a knee replacement femoral component with a portion removed to show internal structure.
Fig. 3 is a top perspective view of the knee replacement femoral component shown in Fig. 2.
Fig. 4 is a perspective view of the knee replacement femoral component shown in Fig. 2 in use in a patient as a replacement knee component
Fig. 5 is a perspective view of a second example of a cast zirconium alloy substrate defining a hip replacement femoral head with a portion removed to show internal structure.
Fig. 6 is a perspective view of the hip replacement femoral head in use in a patient as a replacement hip.
Fig. 7 is a flow diagram depicting a method of forming an oxide layer on a metal body, wherein the metal body is produced via Metal Injection Molding (MIM).

### Detailed Description

The disclosed methods will become better understood through review of the following detailed description in conjunction with the figures. The detailed description and figures provide mere examples of the various inventions described herein. Those skilled in the art will understand that the disclosed examples may be varied, modified, and altered without departing from the scope of the inventions described herein. Many variations are contemplated for different applications and design considerations; however, for the sake of brevity, each and every contemplated variation is not individually described in the following detailed description.

Throughout the following detailed description, examples of various methods are provided. Related features in the examples may be identical, similar, or dissimilar in different examples. For the sake of brevity, related features will not be redundantly explained in each example. Instead, the use of related feature names will cue the reader that the feature with a related feature name may be similar to the related feature in an example explained previously. Features specific to a given example will be described in that particular example. The reader should understand that a given feature need not be the same or similar to the specific portrayal of a related feature in any given figure or example.

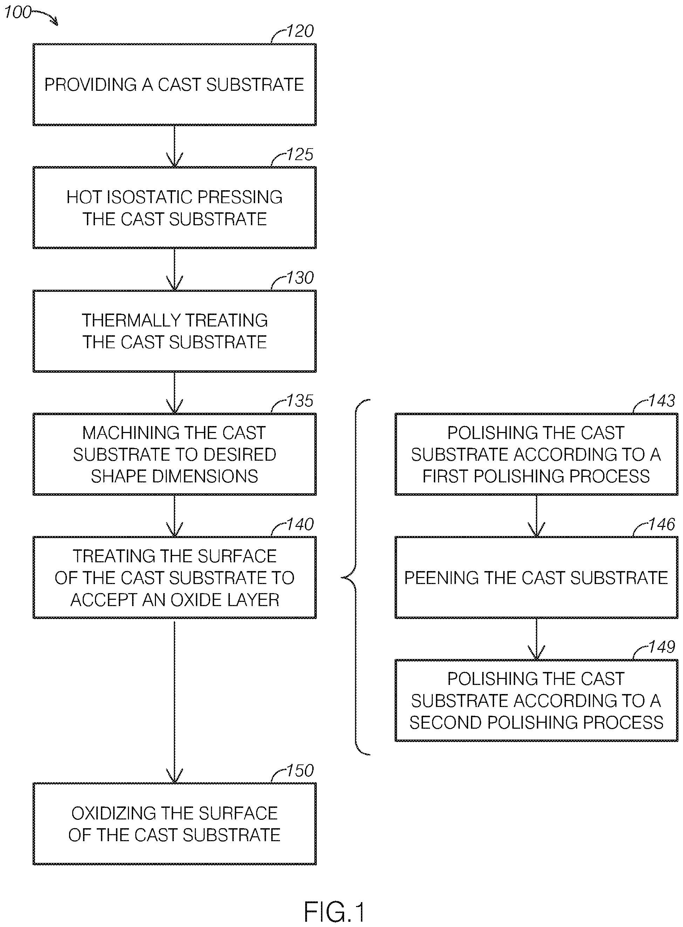

With reference to Fig. 1, a first example of a method for preparing a surface of a cast zirconium alloy substrate for oxidation and oxidizing the surface of said substrate, method 100, will now be described. Method 100 includes providing a cast substrate at step 120, hot isostatic pressing the cast substrate at step 125, thermally treating the cast substrate at step 130, machining the cast substrate to desired shape dimensions at step 135, treating the surface of the cast substrate to accept an oxide layer at step 140, and oxidizing the surface of the cast substrate at step 150.

Method 100 and other disclosed methods overcome many of the shortcomings of conventional processes of producing zirconium alloy products. In particular, method 100 improves the oxide layers of cast zirconium oxide parts such that they may serve as cost-effective alternatives to their forged analogues. These improvements are accomplished, for example, through a series of steps that modify the surface microstructure of cast parts to encourage the surface to accept a satisfactory oxide layer.

The steps to modify the surface microstructure may include a series of heat treating, pressure treating, and surface finishing steps. These steps, collectively and individually, may reduce surface imperfections, such as wide, visible grain boundaries or irregular or coarse surface characteristics. By repairing these imperfections on cast parts, these steps may, when applied, encourage cast parts to accept more satisfactory oxide layers than untreated cast parts. Further, some examples may result in oxidized cast parts with a desired aesthetic blue-black or black appearance free of visible grain boundaries, as is often found on oxidized zirconium-2.5 niobium forgings.

The improved surface characteristics, such as grain size and smoothness, of cast parts produced from method 100 encourage the casts to more satisfactorily accept zirconium oxide layers at step 150. The resulting layers may be more dense, smooth, and uniform than those accepted by many conventional cast parts. By using the disclosed methods, cast parts may serve as a cost-effective alternative to forged analogues in providing near-shape, ASTM-compliant zirconium oxide parts for medical implants.

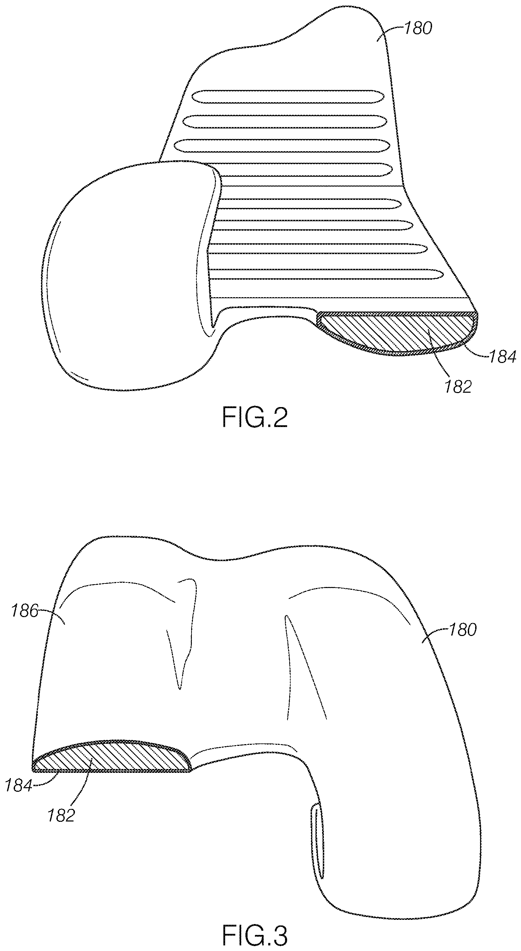

The disclosed methods may be particularly suited to prosthetic implants, such as femoral knees and hips. For example, Figs. 2-6 show two examples of prosthetic implants which have been treated and oxidized by methods disclosed herein to produce a satisfactory zirconium oxide layer: femoral head 160 and femoral knee 180. Because these illustrated examples have been treated and oxidized according to the method steps described in detail below, they include a femoral head oxidized layer 164 and a femoral knee oxidized layer 184. The untreated and oxidized cast parts, or substrates, would be similar in shape and size, but lack the associated oxidized layer.

While this disclosure considers, as examples, cast parts specifically used for medical implants, the disclosed methods have widespread applicability to any use of zirconium oxide alloy parts. In particular, the methods described below may be used with a wide variety of cast parts that benefit from the corrosion and wear resistance characteristics provided by a zirconium oxide layer.

As Fig. 1 illustrates, a cast substrate is provided at step 120. In some examples, the substrate is a cast metal, near-shape part The cast substrates may, in some examples, define cast medical implant parts made of a zirconium alloy, such as Zirconium-2.5 Niobium.

Figs. 2-6 show two examples of such cast parts, femoral head 160 and femoral knee 180 (albeit in post-treated and oxidized conditions defining, respectively, femoral head oxidized layer 164 and femoral knee oxidized layer 184). Although Figs. 2-6 provide two particular cast shapes to which the disclosed methods may be applied, the disclosed methods may include providing cast substrates in different forms than those shown in Figs. 2-6.

In some examples, the casts provided may have been cast to near-shape parts using centrifugal casting processes. Centrifugal casting may provide improved pre-treatment surface conditions compared to alternative casting methods. Centrifugally casted casts are not, however, required. In fact, disclosed methods may improve the surface quality of lower quality casts to cause the casts to adequately accept satisfactory zirconium oxide layers.

As Fig. 1 shows, the cast substrate is hot isostatic pressed at step 125. Hot isostatic pressing often includes placing the cast in a chamber surrounded by an inert fluid, which often is a gas. The inert gas applies a substantially even, predetermined pressure around the entire exposed surface of the part being pressed.

Applying pressure evenly effectively reduces the internal porosity of the cast part, improving the part's mechanical properties such as hardness, smoothness, and uniformity, while retaining a substantially similar shape. Improving the part's mechanical properties may at least partially result from increasing the density of the cast part. Relative to a cast part that was not isostatically pressed, the higher density cast part has a reduced porosity and increased material integrity. The pressure applied may be adjusted by introducing or removing inert fluid to or from the chamber or by adjusting the temperature of the contained gas.

In some examples, the casts are isostatically pressed at step 125 at a pressure between 103.4 Newtons per square millimetre to 179.3 Newtons per square millimetre (15,000 to 26,000 pounds per square inch). The inventor has observed that a target pressure of 172.4 Newtons per square millimetre (25,000 psi) has produced especially satisfactory results. However, any pressure between 103.4 Newtons per square millimetre and 179.3 Newtons per square millimetre (15,000 and 26,000 psi) has been observed to produce satisfactory cast parts with reduced internal porosity.

With regard to temperature, the casts may be isostatically pressed at step 125 at a temperature range of 815 degrees Celcius to 926 degrees Celcius (1500 to 1700 degrees Fahrenheit). Hot isostatically pressing the cast part at 898°C (1,650 °F) has been observed to produce excellent results.

In one example, the cast provided at step 120 is hot isostatically pressed at 172.4 Newtons per square millimetre (25,000 psi) at 898 °C (1,650 °F) for a period of 2 hours. Timeframes of 1 to 2 hours at the temperatures and pressures described above are also suitable.

The temperature, pressure, and time parameter examples described above for the hot isostatic pressing step have been found to produce satisfactory results. Skilled technicians will recognize that various combinations of temperature, pressure, and time within the ranges described above will yield satisfactory results.

As Fig. 1 shows, the cast substrate is thermally treated at step 130. In some examples, thermally treating the cast substrate includes exposing the cast part to a temperature of 982 degrees Celcius to 1315 degrees Celcius (1,800 to 2,400 degrees Fahrenheit) for a period of 1 to 5 hours. Exposing the cast to a temperature of over 1093 degrees Celcius (2000 degrees Fahrenheit) for two hours has been found to provide particularly satisfactory results.

Thermally treating the cast in this manner may produce, amongst other benefits, finer, more uniform grain boundaries proximate the surface of the cast and, in some cases, throughout the cast. The thermal treatment may, for example, encourage alloy elements and segregated elements at grain boundaries to diffuse within the cast and evenly redistribute throughout the cast's internal material. As a result, thermally treating the cast may provide it with improved mechanical properties, such as improved toughness and ductility.

In some examples, thermally treating the cast substrate may include a rapid quench step. In this step, the cast is rapidly quenched at two bars of pressure within a lower-temperature quenching medium to rapidly reduce the temperature of the cast The cast may, for example, be reduced from 1093 degrees Celcius (2000 degrees Fahrenheit) to below 93 degrees Celcius (200 degrees Fahrenheit) in a short period of time in a quenching medium that is 65 degrees Celcius (150 degrees Fahrenheit) or less. In some examples, quenching the cast to reduce its temperature to 65 degrees Celcius (150 degrees Fahrenheit) has been found to produce particularly satisfactory results.

Rapid quenching as described above has been observed to more quickly reduce atomic movement within the cast substrate thereby reducing the amount of time required to reset the cast's microstructure. Resetting the microstructure more quickly may result in finer, more uniform grain boundaries compared to ambient cooling.

As Fig. 1 illustrates, the cast substrate is machined at step 135. Machining the cast substrate at step 135 includes conforming the cast substrate to desired dimensions for the part after it is treated at step 125 or step 130. In some cases, step 125 and/or step 130 may introduce flaws in the cast's shape or size. Machining at step 135 may, for example, help correct any such introduced flaws.

As Fig. 1 shows, the surface of the cast substrate is treated to accept an oxide layer at step 140. In some examples, treating the surface of the cast substrate may include three sub-steps: polishing the cast substrate according to a first polishing process at step 143, peening the cast substrate at step 146, and polishing the cast substrate according to a second polishing process at step 149. The surface treatments applied at step 140 further improve surface characteristics of the cast to increase the likelihood of satisfactory oxidation at step 150.

As Fig. 1 illustrates, the cast substrate is polished according to a first polishing process at step 143. This first polishing process may include a vibratory finishing step, wherein one or more casts may be placed in a container amongst polishing media, wherein the casts and the polishing media are tumbled relative one another. In some examples, this vibratory finishing step is repeated to perform a multi-stage vibratory finishing process, wherein the cast is tumbled within abrasive particle media of progressively smaller or of increasingly fine granularity at each stage.

In some examples, this multi-stage vibratory finishing process may include five stages. Examples of granularities used in such a five-stage process may include: A-80, A-30, A-16, A-6, and 2400 grit. In some examples, this vibratory finishing process may be referred to as "harperizing" performed according to "harperizing processes" known in the art.

As Fig. 1 shows, the cast substrate is peened at step 146. In some examples, peening the cast substrate includes repeatedly contacting the cast's surface with one or more articles of peening media to alter and refine the cast's surface's microstructure without altering the substrate material. Prior to peening casts' surfaces, the surfaces may define large, visible exterior grain boundaries, which may reduce the surfaces' ability to accept a satisfactory oxide layer. In some examples, peening improves the casts' surface characteristics and, in some examples, treats the surface to define visual and structural uniformities of equal or greater quality than forged parts.

In some examples, the cast is peened by repeatedly blasting spherical glass beads toward the cast's surface at approximately 0.69 Newtons per square millimetre (100 pounds per square inch). Additionally or alternatively, steel beads, ceramic beads, and/or steel shot may be used as the peening media. In some examples, casts are sufficiently peened after repeatedly blasting peening media toward the cast substrate for up to five minutes. However, peening the surface for 30 seconds to 60 seconds has been found to provide satisfactory results in many cases.

By repeatedly blasting the cast's surface with the peening media, the surface layer of the cast is plastically deformed as a result of the compressive force applied by the peening media. This plastic deformation caused by the peening media's compressive force is substantially permanent, and thus should not return elastically to its original lattice microstructure (as often occurs with elastic deformation). This may result in a permanent change to the microstructure of the surface layer, effectively defining a "matte" or "blended-in" appearance of the grain boundaries that presents well defined or visually indistinguishable lines.

As Fig. 1 illustrates, the cast substrate is polished according to a second polishing process, or a finishing process, at step 149. This second process includes a multi-stage vibratory polishing step substantially similar to the one performed at step 143. The second polishing process, however, additionally or alternatively may include a final grinding step.

In the final grinding step, a fine polishing compound is applied to the cast's surface via a contacting-type grinding machine. In some examples, this contacting-type grinding machine applies the polishing compound to the cast's surface to remove from 0.001 to 0.002 inches of thickness from the surface, resulting in substantially smooth, bright surfaces that define a reduced number of scratches and visible grain boundary lines. In some examples, "finishing the peened surface" may be accomplished by performing the multi-stage vibratory polishing step, the final grinding step, or both of these steps.

Upon polishing the cast substrate according to the second polishing or finishing process at step 149, the cast substrate has been prepared for oxidation. As a result, some of the disclosed methods may end at this step. For example, many manufacturers may desire to simply produce cast parts treated to accept oxidation without performing an active oxidation step. Similarly, this disclosure notes that the improvements in surface properties provided by the foregoing steps, independently or cooperatively, may reduce apparent grain boundaries and produce substantially smooth, dense, and uniform cast products with low friction coefficients, which may be beneficial without an further oxidation process.

As Fig. 1 shows, the surface of the cast substrate is oxidized at step 150. At this step, the treated surface of a cast accepts an oxide layer defining a thin, transparent oxygen rich film. These natural films, particularly at thicknesses of 2-10 microns, provide hard, uniform, smooth, and dense surfaces, particularly in examples wherein cast substrates define zirconium alloy parts treated according to the disclosed methods. The oxide layers provide many of the desired surface characteristics discussed above, which may be particularly useful with medical implants. Additionally or alternatively, transparent, natural films often produce bright metallic appearances, which may, in some examples, appear blue-black or black in color.

In various examples, oxidizing the surface of the cast substrate may be accomplished either actively or passively, or a combination thereof. Zirconium-2.5 niobium, for example, oxidizes when exposed to air, with or without further intervention. However, cast substrates may be exposed to heat for a predetermined length of time to accelerate the oxidization process, which may produce harder, smoother, and more uniform oxidized layers in reduced time compared to passively oxidizing a cast substrate. Heating zirconium-2.5 niobium casts to 315-815 degrees Celcius (600-1500 degrees Fahrenheit) in an oxidative environment for a period of 2-6 hours has been found to produce particularly satisfactory results.

The resultant oxidized casts often present a blue-black or black visual appearance and lack visible grain boundary lines. In some examples, the appearance may be similar to (or even indistinguishable from) oxidized wrought or forged zirconium-2.5 niobium medical implants. Further, the resultant oxidized casts define surface characteristics, such as low friction coefficients, increased hardness, and resistance to wear and corrosion, that may equal or exceed those of wrought or forged oxidized zirconium-2.5 niobium medical implants.

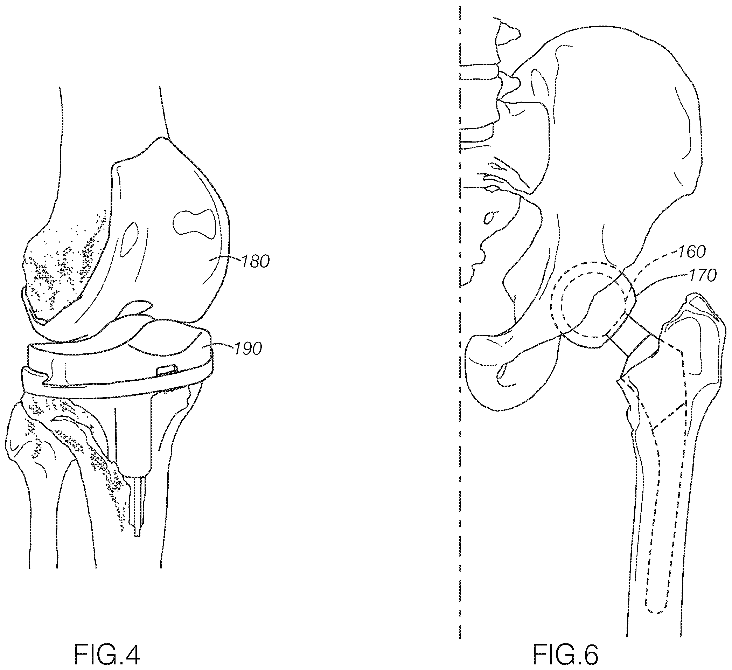

Figs. 2-6 provide two examples of cast parts that have been treated and oxidized according to disclosed methods: femoral head 160 and femoral knee 180. As Fig. 2 shows, femoral knee 180 defines femoral knee oxidized layer 184 surrounding an internal zirconium oxide layer 182, which provides desired surface characteristics to femoral knee 180. For example, femoral knee 180's upper surface 186 defines a blue-black metallic sheen (though the color is not readily displayed in the figure) that defines a substantially hard, smooth, uniform shape. As Fig. 4 shows, femoral knee 180 is appropriately shaped to serve as a knee replacement femoral element, wherein upper surface 186 is configured to articulate against a paired knee member 190.

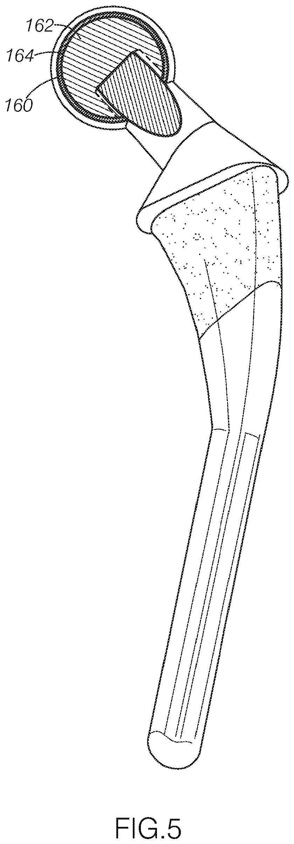

As Fig. 5 shows, femoral head 160 similarly defines femoral head oxidized layer 164 enclosing an internal zirconium oxide layer 162. Similar to femoral knee oxidized layer 184, femoral head oxidized layer 164 is configured to articulate within a femoral head receiving member 170 when implanted in hip replacement, illustrating the importance of femoral head oxidized layer 164's smooth, uniform, low friction design. Although not discussed at length, this disclosure contemplates paired knee members, similar to paired knee member 190, and femoral head receiving members, such as femoral head receiving member 170, being cast, treated, and oxidized according to disclosed methods additionally or alternatively to the femoral heads and knees. In other examples, however, paired knee members and femoral head receiving members may be constructed out of a soft plastic.

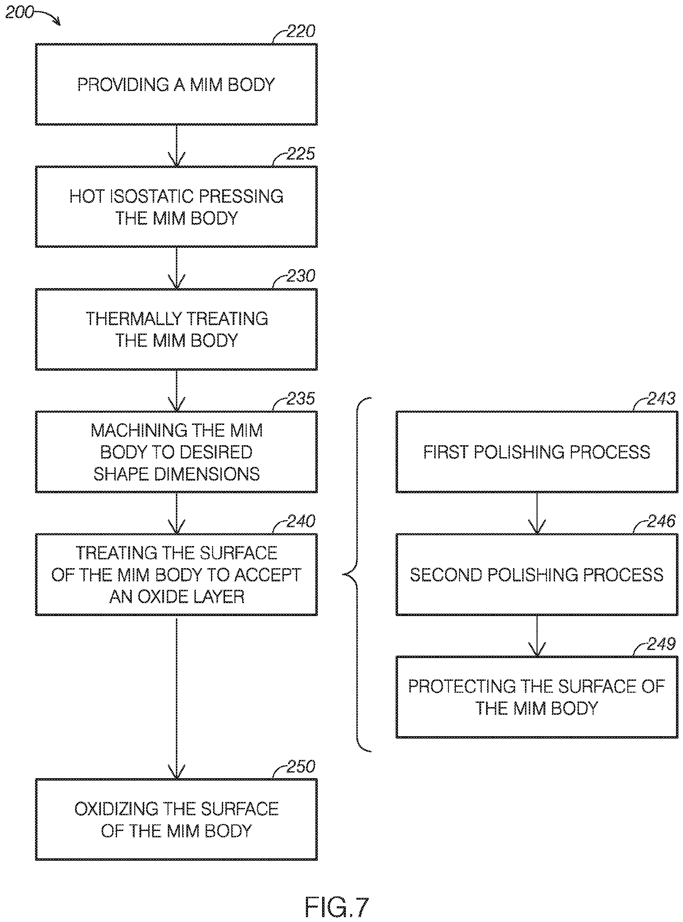

Turning now to Fig. 7, a flow diagram depicting a method 200 of forming an oxide layer on a metal body is shown. According to the invention, the metal body is produced via Metal Injection Molding (MIM). Method 200 includes many similar or identical steps to Method 100. Thus, for the sake of brevity, each step of method 200 will not be redundantly explained. Rather, key distinctions between method 200 and method 100 will be described in detail and the reader should reference the discussion above for features substantially similar between the two methods.

As shown in Fig. 7, method 200 includes the steps of providing 220 a MIM (Metal Injection Molded) body; hot isostatic pressing 225 the MIM body, heat treating 230 the MIM body, machining 235 the MIM body to desired shape dimensions, treating 240 the surface of the MIM body; and oxidizing 250 the surface of the MIM body. Treating 240 the surface of the MIM body includes a first polishing step 243, and second polishing step 246 and protecting 249 the surface of the MIM body prior to oxidizing the surface.

The treating the surface step 240 may include any of the steps described above with respect to surface treatment step 140. For example, the surface treatment step may include a multistage vibratory finishing process. In some embodiments, the MIM body may be processed via particle media that is increasingly fine at each stage. For example, the MIM body may be tumbled in particle media that is increasingly fine at each stage.

As shown in Fig. 7, the surface treatments step includes a first polishing step 243, and second polishing step 246. In some embodiments, the first polishing step 243 may be a first vibratory finishing step. In some embodiments, the second polishing step may be a second vibratory finishing step. In some embodiments, at least one of the first and second vibratory finishing steps comprises a harperizing process. Furthermore, in some embodiments, at least one of the first and second vibratory finishing steps comprises a multi-stage vibratory finishing process.

The MIM body is produced via Metal Injection Molding (MIM). Metal Injection Molding the MIM body may include mixing a feedstock comprising one or more metal powders and one or more binders. The feedstock may then be injected into a mold to produce a "green part" of near shape dimensions. The resulting green part may be removed from the mold.

In some embodiments, the green part may then be placed in a second mold. A second feedstock comprising one or more metal powders and one or more binders may be mixed. The second feedstock may then be injected into the second mold, thereby forming a dual composition green part having an over-molded portion made up of the second feedstock and a base portion made up of the first feedstock.

At least some of the binder(s) may then be removed, for example by submerging the green part in a solvent bath. Next the green part may be sintered, thereby sintering the metal powder(s) together to form the MIM body. In this regard, MIM bodies comprising a base portion of a first material and an over-molded portion of a second material may be produced. As can be appreciated, a MIM body comprising a second over-molded portion of a third material could also be produced via a third mixing/molding step. The MIM body may then be processed as described above with respect to method 200.

As described above zirconium alloys may be particularly well suited to the disclosed oxidation layer formation processes. Thus, in some embodiments, the first MIM body may comprise a zirconium alloy. For example, the MIM body may comprise a zirconium-niobium alloy, such as zirconium-2.5 niobium, among others.

As described above, in some embodiments, the MIM body may have a dual composition, e.g., a base portion of a first material and an over-molded portion of a second material. In some embodiments, the base portion may comprise a titanium alloy. For example, the base portion may comprise a Ti-6Al-4V alloy base. In some embodiments, the over-molded portion may comprise any of the zirconium alloys described above.

As described above, the MIM body may be hot isostatic pressed 225. IN one embodiment, the Metal Injection Molded body may be hot isostatic pressed at a pressure range of 103.4 Newtons per square millimetre to 179.3 Newtons per square millimetre (15,000 pounds per square inch to 26,000 pounds per square inch). In some embodiments, the Metal Injection Molded body may be hot isostatic pressed at a temperature of 815 degrees Celsius to 926 degrees Celsius (1,500 degrees Fahrenheit to 1,700 degrees Fahrenheit) for a period of one to four hours.

As described above, the MIM body may be heat treated 230. In some embodiments, the heat treating step 230 may comprise heating the Metal Injection Molded body to 982 degrees Celsius to 1315 degrees Celsius (1,800 degrees Fahrenheit to 2,400 degrees Fahrenheit) for a period of one to five hours.

As described above, the surface of the MIM body may be oxidized 250. In some embodiments, the oxidizing step may include heating the substrate to 300 to 815 °C (600 to 1,500 °F) in an oxidative environment for 2 to 6 hours. In some embodiments, the oxidizing step may include forming a zirconium oxide surface layer having a thickness of 2 to 10 microns.

In some embodiments, after the oxidizing step 250, the oxidized surface may then be mechanically finished. In some embodiments, the mechanically finishing step may comprise grinding the oxidized surface to remove up to 0.05 mm (0.002") of surface material.

The disclosure above encompasses multiple distinct inventions with independent utility. While each of these inventions has been disclosed in a particular form, the specific embodiments disclosed and illustrated above are not to be considered in a limiting sense as numerous variations are possible.

## Claims

1. A method of forming an oxide layer on a surface of a Metal Injection Molded body, wherein the surface comprises a zirconium alloy, the method comprising:
hot isostatic pressing (225) the Metal Injection Molded body;
heat treating (230) the Metal Injection Molded body;
machining (235) the Metal Injection Molded body to desired shape dimensions;
polishing (240) the surface of the Metal Injection Molded body, wherein the polishing comprises:
a first vibratory finishing step (243); and
a second vibratory finishing step (249); and
oxidizing (250) the polished surface of the Metal Injection Molded body, **characterised in that** the Metal Injection Molded body comprises a base portion and an over-molded portion, wherein the base portion is a titanium alloy and the over-molded portion is the zirconium alloy.

2. The method of claim 1, wherein the Metal Injection Molded body is hot isostatic pressed at a pressure range of 103.4 Newtons per square millimetre to 179.3 Newtons per square millimetre (15,000 pounds per square inch to 26,000 pounds per square inch) at a temperature of 815 degrees Celcius to 926 degrees Celcius (1,500 degrees Fahrenheit to 1,700 degrees Fahrenheit) for a period of one to four hours.

3. The method of claim 1, wherein heat treating the Metal Injection Molded body includes heating the body to a temperature of 982 degrees Celcius to 1315 degrees Celcius (1,800 degrees Fahrenheit to 2,400 degrees Fahrenheit) for a period of one to five hours.

4. The method of claim 1, wherein the zirconium alloy comprises a zirconium-niobium alloy.

5. The method of claim 4, wherein the zirconium-niobium alloy is zirconium-2.5 niobium.

6. The method of claim 1, wherein oxidizing the polished surface includes forming a zirconium oxide surface layer having a thickness of 2 to 10 microns.

7. The method of claim 1, wherein oxidizing the polished surface includes heating the Metal Injection Molded body to a temperature of 315 degrees Celcius to 815 degrees Celcius (600 degrees Fahrenheit to 1,500 degrees Fahrenheit) in an oxidative environment for 2 to 6 hours.

8. The method of claim 1, further comprising mechanically finishing the oxidized surface.

9. The method of claim 1, wherein mechanically finishing the oxidized surface includes grinding to remove up to 0.05 mm (0.002") of surface material.

10. The method of claim 1, wherein at least one of the first and second vibratory finishing steps comprises a harperizing process.

11. The method of claim 1, wherein at least one of the first and second vibratory finishing steps comprises a multi-stage vibratory finishing process.

## Patentansprüche

1. Ein Verfahren zur Bildung einer Oxidschicht auf einer Oberfläche eines Metallspritzgusskörpers, wobei die Oberfläche eine Zirconiumlegierung umfasst und das Verfahren umfass:
Isostatisches Heißpressen (225) des Metallspritzgusskörpers;
Hitzebehandeln (230) des Metallspritzgusskörpers;
Bearbeiten (235) des Metallspritzgusskörpers zu gewünschten Formabmessungen;
Polieren (240) der Oberfläche des Metallspritzgusskörpers, wobei das Polieren umfasst:
einen ersten Vibrationsendbearbeitungsschritt (243); und
einen zweiten Vibrationsendbearbeitungsschritt (249); und
Oxidieren der polierten Oberfläche des Metallspritzgusskörpers,
**dadurch gekennzeichnet, dass** der Metallspritzgusskörper einen Basisabschnitt und einen umspritzen Abschnitt umfasst, wobei der Basisabschnitt eine Titanlegierung und der umspritze Abschnitt die Zirconiumlegierung ist.

2. Verfahren nach Anspruch 1, wobei der Metallspritzgusskörper bei einem Druck im Bereich von 103,4 N/mm² bis 179,3 N/mm² (15.000 Pfund/Inch² bis 26.000 Pfund/Inch²) bei einer Temperatur von 815°C bis 926°C (1.500°F bis 1.700°F) für eine Zeitspanne von ein bis vier Stunden isostatisch heißgepresst wird.

3. Verfahren nach Anspruch 1, wobei die Hitzebehandlung des Metallspritzgusskörpers eine Erhitzung des Körpers auf eine Temperatur von 982°C bis 1315°C (1.800°F bis 2.400°F) für eine Zeitspanne von ein bis fünf Stunden umfasst.

4. Verfahren nach Anspruch 1, wobei die Zirconiumlegierung eine Zirconium-Nioblegierung umfasst.

5. Verfahren nach Anspruch 4, wobei die Zirconium-Nioblegierung Zirconium-2.5 Niob ist.

6. Verfahren nach Anspruch 1, wobei das Oxidieren der polierten Oberfläche die Bildung einer Zirconiumoxidoberfächenschicht aufweisend eine Dicke von 2 bis 10 µm umfasst.

7. Verfahren nach Anspruch 1, wobei das Oxidieren der polierten Oberfläche das Erhitzung des Metallspritzgusskörpers auf eine Temperatur von 315°C bis 815°C (600°F bis 1.500°F) in einer oxidativen Umgebung für 2 bis 6 Stunden umfasst.

8. Verfahren nach Anspruch 1, weiterhin umfassend eine mechanische Endbearbeitung der oxidierten Oberfläche.

9. Verfahren nach Anspruch 1, wobei die mechanische Endbearbeitung der oxidierten Oberfläche Schleifen zur Entfernung von bis zu 0,05 mm (0,002") Oberflächenmaterial umfasst.

10. Verfahren nach Anspruch 1, wobei mindestens einer der ersten und zweiten Vibrationsendbearbeitungsschritte einen Harperisierungprozess umfasst.

11. Verfahren nach Anspruch 1, wobei mindestens einer der ersten und zweiten Vibrationsendbearbeitungsschritte einen mehrstufigen Vibrationsendbearbeitungsprozess umfasst.

## Revendications

1. Procédé de formation d'une couche d'oxyde sur une surface d'un corps métallique moulé par injection, dans lequel la surface comprend un alliage de zirconium, le procédé comprenant :
un pressage isostatique à chaud (225) du corps métallique moulé par injection ;
un traitement à la chaleur (230) du corps métallique moulé par injection ;
un usinage (235) du corps métallique moulé par injection à des dimensions de forme souhaitées ;
un polissage (240) de la surface du corps métallique moulé par injection, lequel polissage comprend :
une première étape de vibroabrasion (243) ; et
une deuxième étape de vibroabrasion (249) ; et
une oxydation (250) de la surface polie du corps métallique moulé par injection,
**caractérisé en ce que** le corps métallique moulé par injection comprend une partie de base et une partie surmoulée, la partie de base étant un alliage de titane et la partie surmoulée étant l'alliage de zirconium.

2. Procédé selon la revendication 1, dans lequel le corps métallique moulé par injection est pressé par pressage isostatique à chaud à une pression située dans la plage allant de 103,4 Newtons par millimètre carré à 179,3 Newtons par millimètre carré (de 15 000 livres par pouce carré à 26 000 livres par pouce carré) à une température de 815 degrés Celsius à 926 degrés Celsius (de 1 500 degrés Fahrenheit à 1 700 degrés Fahrenheit) pendant une période d'une à quatre heures.

3. Procédé selon la revendication 1, dans lequel le traitement à la chaleur du corps métallique moulé par injection comprend un chauffage du corps à une température de 982 degrés Celsius à 1 315 degrés Celsius (de 1 800 degrés Fahrenheit à 2 400 degrés Fahrenheit) pendant une période d'une à cinq heures.

4. Procédé selon la revendication 1, dans lequel l'alliage de zirconium comprend un alliage de zirconium-niobium.

5. Procédé selon la revendication 4, dans lequel l'alliage de zirconium-niobium est le zirconium-2,5 niobium.

6. Procédé selon la revendication 1, dans lequel l'oxydation de la surface polie comprend la formation d'une couche de surface en oxyde de zirconium ayant une épaisseur de 2 à 10 micromètres.

7. Procédé selon la revendication 1, dans lequel l'oxydation de la surface polie comprend le chauffage du corps métallique moulé par injection à une température de 315 degrés Celsius à 815 degrés Celsius (de 600 degrés Fahrenheit à 1 500 degrés Fahrenheit) dans un environnement oxydant pendant 2 à 6 heures.

8. Procédé selon la revendication 1, comprenant en outre le finissage mécanique de la surface oxydée.

9. Procédé selon la revendication 1, dans lequel le finissage mécanique de la surface oxydée comprend un meulage pour retirer jusqu'à 0,05 mm (0,002") de matériau de surface.

10. Procédé selon la revendication 1, dans lequel au moins l'une des première et deuxième étapes de vibroabrasion comprend un traitement de harpérisation.

11. Procédé selon la revendication 1, dans lequel au moins l'une des première et deuxième étapes de vibroabrasion comprend un traitement de vibroabrasion à étapes multiples.
